# EUROPEAN PATENT APPLICATION

(11) **EP 4 663 186 A1**
(43) Date of publication of application: **17.12.2025**
(21) Application number: 25182107.0
(22) Date of filing: 11.06.2025
(51) Int. Cl.: A61K 9/20, A61K 31/4545

(54) **A TABLET COMPOSITION COMPRISING BENIDIPINE**

(30) Priority: 14.06.2024 TR 2024007785
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); GULER, Tolga, Istanbul (TR); SUEKINCI, Sinan, Istanbul (TR)
(74) Representative: Genç Ilhan, Oznur

(57) **Abstract**

The present invention relates to a tablet composition comprising benidipine or pharmaceutical acceptable salt thereof, polyvinyl alcohol and at least one disintegrant, wherein the amount of disintegrant is between 0.1% and 10.0% by weight in the total composition. The present invention also relates to a simple, rapid, cost-effective, time-saving and industrially suitable process for the preparation of a tablet composition.

## Description

### Field of the Invention

The present invention relates to a tablet composition comprising benidipine or pharmaceutical acceptable salt thereof, polyvinyl alcohol and at least one disintegrant, wherein the amount of disintegrant is between 0.1% and 10.0% by weight in the total composition. The present invention also relates to a simple, rapid, cost-effective, time-saving and industrially suitable process for the preparation of a tablet composition.

### Background of the Invention

Benidipine, chemically known as 1,4-dihydro-2,6-dimethyl-4-(3-nitrophenyl)-3,5-pyridinedicarboxylic acid methyl 1-(phenylmethyl)-3-piperidinyl ester hydrochloride, has the empirical formula of C₂₈H₃₁N₃O₆ - HCl and molecular weight of 542.03. Benidipine has the following chemical structure of Formula I.

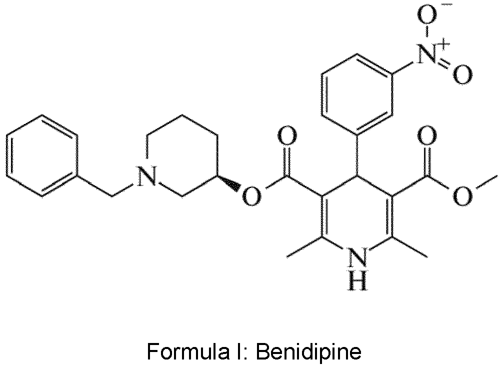

Benidipine is a calcium channel blocker useful for treatment of hypertension, renal parenchymal hypertension and angina pectoris. Benidipine is commercially available under the tradename Coniel(R) Tablets as the hydrochloride salt of benidipine in strengths of 2 mg, 4 mg and 8 mg, and generally prescribed in regimes of once or twice daily.

Benidipine hydrochloride is an odorless yellow crystalline powder. Benidipine is a class II drug in the BCS classification, that is, poorly water soluble. For poorly soluble drugs, its dissolution is the rate-limiting process of absorption and is often the most important factor affecting its bioavailability. It is very soluble in formic acid, freely soluble in dimethylformamide, soluble in methanol or ethanol, slightly soluble in acetic anhydride and relatively insoluble in water.

The patent WO2004110448A1 disclose the invention benidipine hydrochloride composition which has the property of enabling rapid dissolution for accelerating the rapid absorption of benidipine hydrochloride as the main drug.

It is known in the prior art that the solubility of benidipine is poor and there are patent applications written regarding this. Additionally, since benidipine HCl is present in low amounts in the formulation, its mixing and formulation can be very challenging due to problems with content uniformity and stability. Therefore, a new formulation with the desired solubility, stability and content uniformity is still needed.

### Detailed Description of the Invention

The main object of the present invention is to obtain a tablet composition comprising benidipine or pharmaceutical acceptable salt thereof which has the desired dissolution profile, stability and content uniformity.

Another object of the present invention is to provide an effective process for a tablet comprising benidipine or pharmaceutical acceptable salt thereof which is characterized by excellent pharmacotechnical properties, such as flowability and compressibility.

In the prior art, poor solubility of benidipine and low amounts of benidipine HCl in the formulation were found to be very difficult to mix and formulate due to content homogeneity and stability issues. We surprisingly found that the use of benidipine in combination with polyvinyl alcohol and the use of low amounts of disintegrant provided stabilty, the desired dissolution profile and content uniformity.

According to one embodiment of the invention a tablet composition comprising benidipine and polyvinyl alcohol and at least one disintegrant, wherein the amount of disintegrant is between 0.1% and 10.0% by weight in the total composition.

According to one embodiment of the present invention, the amount of benidipine hydrochloride is between 0.1% and 10.0% by weight in the total composition. Preferably, it is between 1.0% and 7.0% by weight in the total composition.

According to one embodiment of the present invention, the amount of polyvinyl alcohol is between 0.01% and 8.0% by weight in the total composition. Preferably, it is between 0.1% and 5.0% by weight in the total composition. The use of polyvinyl alcohol at this ratio provided the desired dissolution profile.

Suitable disintegrants are selected from the group comprising pregelatinized starch, crospovidone, croscarmellose sodium, starch, low-substituted hydroxypropyl cellulose, sodium carboxymethyl cellulose, calcium carboxymethyl cellulose, carboxymethyl cellulose, docusate sodium, low substituted hydroxypropyl cellulose, sodium alginate, corn starch, sodium starch glycolate, sodium glycine carbonate or mixtures thereof.

According to one embodiment of the present invention, the disintegrant is pregelatinized starch.

According to one embodiment of the present invention, the amount of disintegrants is between 0.1% and 10.0% by weight in the total composition. Preferably, it is between 1.0% and 8.0% by weight in the total composition. Preferably, it is between 2.5% and 6.0% by weight in the total composition. The use of disintegrants at these ratios provided stability and content uniformity.

According to one embodiment of the invention, a tablet composition comprises at least one pharmaceutically acceptable excipient which is selected from the group comprising fillers, lubricants, coating agents or mixtures.

Suitable fillers are selected from the group comprising lactose monohydrate, microcrystalline cellulose, lactose, mannitol, spray-dried mannitol, dextrose, sucrose, fructose, maltose, sorbitol, xylitol, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to one embodiment of the present invention, the filler is lactose monohydrate.

According to one embodiment of the present invention, the amount of fillers is between 75.0% and 95.0% by weight in the total composition. Preferably, it is between 78.0% and 88.0% by weight in the total composition. The amount provides the desired flowability and compressibility.

Suitable lubricants are selected from the group comprising magnesium stearate, sodium stearyl fumarate, talc, colloidal silicon dioxide, corn, calcium stearate, zinc stearate, magnesium lauryl sulfate, sodium oleate, sodium acetate, sodium benzoate, stearic acid, fumaric acid, glyceryl palmito sulphate or mixtures thereof.

According to one embodiment of the present invention, the lubricant is magnesium stearate.

According to one embodiment of the present invention, the amount of lubricant is between 0.01% and 5.0% by weight in the total composition. Preferably, it is between 0.1% and 3.0% by weight in the total composition.

According to an embodiment of the present invention, the coating agents are lactose monohydrate, hydroxypropyl methylcellulose, titanium dioxide, polyethylene glycol, yellow iron oxide, triacetin, ponceau, FD&C Blue.

According to an embodiment of the present invention, the amount of coating agents is 1.0% to 5.0% by weight by weight in the total composition.

As used herein, 'particle size' means the cumulative volume size distribution as tested by any conventionally accepted method such as the laser diffraction method (i.e. Malvern analysis). The term d (0.9) means, the size at which 90% by volume of the particles are finer. The term d (0.5) means, the size at which 50% by volume of the particles are finer.

According to this embodiment of the present invention, benidipine in the form of the free base or in the form of pharmaceutically acceptable salts has a d (0.9) particle size less than 35 µm, preferably less than 25 µm, preferably less than 18 µm.

According to this embodiment of the present invention, benidipine in the form of the free base or in the form of pharmaceutically acceptable salts has a d (0.9) particle size between 1 µm and 18 µm.

According to one embodiment of the present invention, a tablet composition comprises;
- Benidipine hydrochloride having a d (0.9) particle size less than 35 µm
- Lactose monohydrate
- Pregelatinized starch
- Polyvinyl alcohol
- Magnesium stearate

### Example 1;

| **Ingredients** | **% by weight of the total tablet** | **% by weight of the total tablet** |
|---|---|---|
| Benidipine hydrochloride | 4 | 0.1-10 |
| Lactose monohydrate | 84.5 | 75-95 |
| Pregelatinized starch | 5 | 0.1-10 |
| Polyvinyl alcohol (PVA) | 2.5 | 0.01-8 |
| Magnesium stearate | 1 | 0.01-5 |
| **Tablet weight** | 97 | 95-99 |
| Film coating | 3 | 1-5 |
| · *Opadry II Yellow 33G22265* | | |
| · *Lactose monohydrate 21.00%* | | |
| · *HPMC 6 cP 40.00%* | | |
| · *Titanium dioxide 24.25%* | | |
| · *PEG 3000 Powder 8.00%* | | |
| · *Yellow iron oxide 0.737%* | | |
| · *Triacetin 6.00%* | | |
| · *Ponceau 4R 0.011%* | | |
| · *FD&C Blue No 2 0.002%* | | |
| **Total tablet weight** | 100 | 100 |

A process for example 1;
a) dissolving PVA in water and obtaining PVA solution,
b) mixing benidipine hydrochloride and lactose monohydrate,
c) granulating the mixture from step (b) with PVA solution,
d) drying the mixture,
e) adding pregelatinized starch to the dried granules and mixing,
f) adding magnesium stearate to the mixture from step (e) and mixing,
g) compressing the prepared mixture to form tablets,
h) coating these tablets with coating agents.

## Claims

1. A tablet composition comprising benidipine and polyvinyl alcohol and at least one disintegrant, wherein the amount of disintegrant is between 0.1% and 10.0% by weight in the total composition.

2. The tablet composition according to claim 1, wherein the amount of benidipine hydrochloride is between 0.1% and 10.0% by weight in the total composition.

3. The tablet composition according to claim 1, wherein the amount of polyvinyl alcohol is between 0.01% and 8.0% by weight in the total composition.

4. The tablet composition according to claim 1, wherein disintegrants are selected from the group comprising pregelatinized starch, crospovidone, croscarmellose sodium, starch, low-substituted hydroxypropyl cellulose, sodium carboxymethyl cellulose, calcium carboxymethyl cellulose, carboxymethyl cellulose, docusate sodium, low substituted hydroxypropyl cellulose, sodium alginate, corn starch, sodium starch glycolate, sodium glycine carbonate or mixtures thereof.

5. The tablet composition according to claim 1 or 4, wherein disintegrant is pregelatinized starch.

6. The tablet composition according to claim 1 or 4 or 5, wherein the amount of disintegrants is between 0.1% and 10.0% by weight in the total composition.

7. The tablet composition according to claim 1, wherein a tablet composition comprises at least one pharmaceutically acceptable excipient which is selected from the group comprising fillers, lubricants, coating agents or mixtures.

8. The tablet composition according to claim 7, wherein fillers are selected from the group comprising lactose monohydrate, microcrystalline cellulose, lactose, mannitol, spray-dried mannitol, dextrose, sucrose, fructose, maltose, sorbitol, xylitol, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

9. The tablet composition according to claim 8, wherein the filler is lactose monohydrate.

10. The tablet composition according to claim 8 or 9, wherein the amount of fillers is between 75.0% and 95.0% by weight in the total composition.

11. The tablet composition according to claim 7, wherein lubricants are selected from the group comprising magnesium stearate, sodium stearyl fumarate, talc, colloidal silicon dioxide, corn, calcium stearate, zinc stearate, magnesium lauryl sulfate, sodium oleate, sodium acetate, sodium benzoate, stearic acid, fumaric acid, glyceryl palmito sulphate or mixtures thereof.

12. The tablet composition according to claim 7 or 11, wherein the lubricant is magnesium stearate.

13. The tablet composition according to claim 11 or 12, wherein the amount of lubricant is between 0.01% and 5.0% by weight in the total composition.

14. The tablet composition according to any preceding claim, wherein benidipine in the form of the free base or in the form of pharmaceutically acceptable salts has a d (0.9) particle size less than 35 µm, preferably less than 25 µm, preferably less than 18 µm.
